# EUROPEAN PATENT SPECIFICATION

(11) **EP 4 066 736 B1**
(45) Date of publication and mention of the grant of the patent: **20.08.2025**
(21) Application number: 20894523.8
(22) Date of filing: 18.11.2020
(51) Int. Cl.: A61B 5/024, A61B 5/00, G06V 40/10, G06V 40/16

(54) **HEART RATE ESTIMATION METHOD AND APPARATUS, AND ELECTRONIC DEVICE APPLYING SAME**
VERFAHREN UND VORRICHTUNG ZUR KALKULATION DER HERZFREQUENZ UND ELEKTRONISCHES GERÄT DAMIT
PROCÉDÉ ET APPAREIL D'ESTIMATION DE FRÉQUENCE CARDIAQUE, ET DISPOSITIF ÉLECTRONIQUE APPLIQUANT LEDIT PROCÉDÉ

(30) Priority: 25.11.2019 CN 201911166001
(43) Date of publication of application: 05.10.2022
(73) Proprietor: ARCSOFT CORPORATION LIMITED, Xihu Dist Hangzhou Zhejiang 310012 (CN)
(72) Inventor: ZHANG, Zhirui, Hangzhou, Zhejiang 310012 (CN); XU, Tianjiao, Hangzhou, Zhejiang 310012 (CN); WANG, Jin, Hangzhou, Zhejiang 310012 (CN)
(74) Representative: Penza, Giancarlo
(86) International application number: PCT/CN2020/129836
(87) International publication number: WO 2021/104129

(56) References cited:
- CN-A- 105 266 787
- CN-A- 105 989 357
- CN-A- 109 480 808
- CN-A- 109 602 412
- CN-A- 109 977 858
- CN-A- 110 236 511
- CN-A- 110 367 950
- CN-A- 110 384 491
- US-A1- 2015 302 158
- US-A1- 2019 171 893

## Description

### Cross-Reference to Related Application

The present application claims the priority of Chinese Application No. 201911166001.2, filed in the Chinese Patent Office on November 25, 2019, and entitled "Heart Rate Estimation Method and Apparatus, and Electronic Device Applying Same".

### Technical Field

The present invention relates to a computer vision technology, especially to a heart rate estimation method and apparatus, and an electronic device applyingsame.

### Background

An electrocardiogram is a technique that uses an electrocardiograph to record, from a body surface, a pattern of point activity changes produced by a heart within each cardiac cycle. Although this method has high accuracy, the instrument is expensive and requires professional operation, such that the equipment is cumbersome, and the use scenarios are extremely limited.

Photo plethysmo graphy (PPG) is a non-invasive measurement method for monitoring blood volume changes in living body tissues by means of photoelectric means. When light of a certain wavelength is irradiated on a skin surface such as between fingers, the light will be captured by a photoelectric receiver in a transmission or reflection manner. During the whole process, the light is weakened due to the absorption by skin, muscles, blood and the like, and thus the light intensity reaching the photoelectric receiver will be reduced. The weakening effect of the skin, the muscles and the like on the light intensity is constant, while the weakening effect of blood in blood vessels on the light will show a pulsatile change with the beating of the heart. When the heart contracts, the blood volume in the blood vessels increases, the absorbed light intensity increases, and the light intensity received by the photoelectric receiver decreases accordingly. When the heart dilates, the condition is opposite. By converting the light intensity into an electrical signal, the change in the volume pulse blood flow can be obtained. This method requires a close contact between a sensor and a fixed human body part, thereby having many restrictions on the use methods of users and use scenarios.

CN 109480808 A discloses to detect heart rate by processing a face image, in particular performing spatial pixel average of the green channel of a skin area image, then calculating interpolation of the facial image with a preset frame number to obtain a time sequence signal containing heart beat information.

US 2015/302158 A1 discloses a video-based system to detect heart rate using motion data to reduce the effects of motion on heart rate detection.

### Summary

Embodiments of the present disclosure provide a heart rate estimation method and apparatus, and an electronic device applying same, so as to at least solve the technical problem in the prior art that heart rate estimation can only be realized in a contact manner.

According to one aspect of the embodiments of the present invention, a heart rate estimation method is provided, including: acquiring a face video; performing face detection on the face video to extract a local face area that is configured to perform heart rate estimation; performing first processing on values of pixel points in the local face area to obtain an initial heart rate signal; performing time-frequency domain conversion on the initial heart rate signal to obtain a frequency domain signal; and performing second processing on the frequency domain signal within a heart rate estimation range to obtain a heart rate estimation value, wherein the step of performing first processing on the values of the pixel points in the local face area to obtain the initial heart rate signalincludes: performing weighted average on the values of the pixel points in the local face area according to weights, and taking the value of weighted average as a luminance signal of the current frame; and constituting the initial heart rate signal by the luminance signal of the current frame and luminance signals of previous N historical frames, and wherein the weight is set according to the position of the pixel point in the local face area, and the closer the pixel point is to an edge position in the local face area, the smaller the weight corresponding to the pixel point is.

Optionally, the heart rate estimation method further includes: combining face key point positioning with face detection to extract the local face area that is configured to perform heart rate estimation.

Optionally, before performing the time-frequency domain conversion on the initial heart rate signal, the heart rate estimation method can further include: performing first denoising processing on the initial heart rate signal in the time domain, wherein the first denoising processing method includes at least one of the following: S-G (Savitzky-Golay) filtering, detrend (Detrend) filtering, moving average filtering, normalization (Normalize) processing, and bandpass (Bandpass) filtering.

Optionally, the step of performing second processing on the frequency domain signal within the heart rate estimation range to obtain theheart rate estimation value includes: performing peak value detection on the frequency domain signal within the heart rate estimation range to obtain peak values; sorting the peak values to obtain a sorting result; calculating a confidence coefficient according to the sorting result; and obtaining the heart rate estimation value according to the confidence coefficient.

Optionally, the step of performing second processing on the frequency domain signal within the heart rate estimation range to obtain theheart rate estimation value includes: performing peak value detection on the frequency domain signal within the heart rate estimation range to obtain a highest peak value; taking the frequency of the highest peak value as main frequency, and calculating the energy of a first-order harmonic and a second-order harmonic corresponding to the main frequency, to obtain an energy calculation value; dividing the energy calculation value by the energy of the remaining frequency other than the main frequency, to obtain a confidence coefficient; and obtaining the heart rate estimation value according to the confidence coefficient.

Optionally, the heart rate estimation method further includes: judging whether a detection time exceeds a second threshold value; and when the detection time exceeds the second threshold value, performing third processing on the frequency domain signal to obtain the heart rate estimation range.

Optionally, the step of performing third processing on the frequency domain signal to obtain the heart rate estimation rangeincludes: continuously selecting consecutive X frames or X seconds of frequency domain signals through a sliding window to obtain a heart rate value, buffering the heart rate value, acquiring the heart rate estimation range by using a deep learning method, repeating the action within M frames or M seconds, and averaging all acquired heart rate estimation ranges to obtain a final heart rate estimation range.

Optionally, the peak values are sorted in a traversal manner in a descending order of the peak values, the highest peak value and a second peak value are selected as the sorting result, and a ratio of the highest peak value to the second peak value is taken as the confidence coefficient.

Optionally, the step of obtaining the heart rate estimation value according to the confidence coefficient includes: comparing the confidence coefficient with a first threshold value to obtain a comparison result; when the confidence coefficientis less than the first threshold value, the comparison result indicates that the frequency domain signal is seriously polluted by noise, then discarding the current result, and detecting the next frame; and when the confidence coefficient is not less than the first threshold value, the comparison result indicates that the frequency domain signal is not polluted by noise or the noise pollution is relatively small, then acquiring the frequency corresponding to the highest peak value as theheart rate estimation value.

Optionally, before performing second processing on the frequency domain signal, the heart rate estimation method further includes: performing second denoising processing on the frequency domain signal, wherein the second denoising processing method includes at least one or more of the following: discrete Fourier transform (DFT), and bandpass (Bandpass) filtering.

According to another aspect of the embodiments of the present invention, a heart rate estimation apparatus is further provided, including: a camera shooting unit, configured to acquire a face video; a detection unit, configured to perform face detection on the face video to extract a local face area that is configured to perform heart rate estimation; a first processing unit, configured to perform first processing on values of pixel points in the local face area to obtain an initial heart rate signal; a conversion unit, configured to perform time-frequency domain conversion on the initial heart rate signal to obtain a frequency domain signal; and a second processing unit, configured to perform second processing on the frequency domain signal within a heart rate estimation range to obtain a heart rate estimation value, wherein the first processing unit is further configured to perform weighted average on the values of the pixel points in the local face area according to weights, is configured to take the value of weighted average as a luminance signal of the current frame, is configured to constitute the initial heart rate signal by the luminance signal of the current frame and luminance signals of previous N historical frames, wherein the weight is set according to the position of the pixel point in the local face area, and the closer the pixel point is to an edge position in the local face area, the smaller the weight corresponding to the pixel point is.

According to another aspect of the embodiments of the present invention, a storage medium is further provided, including a stored program, wherein the program, when running, controls a device where the storage medium is located to execute the heart rate estimation method in any of above embodiments.

According to another aspect of the embodiments of the present invention, an electronic device is further provided, including: a processor; and a memory, configured to store executable instructions of the processor, wherein the processor is configured to execute the heart rate estimation method in any of above embodiments by executing the executable instructions.

In the embodiments of the present invention, the following steps are executed: acquiring the face video; performing face detection on the face video to extract thelocal face area that is configured to perform heart rate estimation; performing first processing on the values of the pixel points in the local face area to obtain the initial heart rate signal; performing time-frequency domain conversion on the initial heart rate signal to obtain the frequency domain signal; and performing second processing on the frequency domain signal within the heart rate estimation range to obtain theheart rate estimation value. Therefore, the technical problem in the related art is solved that heart rate estimation can only be realized in a contact manner.

### Brief Description of the Drawings

The drawings described herein are used for providing a further understanding of the present invention and constitute a part of the present application. Exemplary embodiments of the present invention and descriptions thereof are used for explaining the present invention, but do not constitute improper limitations of the present invention. In the drawings:
Fig. 1 is a flowchart of a first optional heart rate estimation method according to an embodiment of the present invention;
Fig. 2 is a schematic diagram of an optional extracted local face area according to an embodiment of the present invention;
Fig. 3 is a flowchart of a second optional heart rate estimation method according to an embodiment of the present invention;
Fig. 4 is an application scenario diagram of a heart rate estimation method provided according to an embodiment of the present invention; and
Fig. 5 is a structural block diagram of an optional heart rate estimation apparatus according to an embodiment of the present invention.

### Detailed Description of the Embodiments

In order that those skilled in the art can better understand the solutions of the present invention, a clear and complete description of technical solutions in the embodiments of the present invention will be given below, in combination with the drawings in the embodiments of the present invention. Apparently, the embodiments described below are merely a part, but not all, of the embodiments of the present invention. All of other embodiments, obtained by those of ordinary skill in the art based on the embodiments of the present invention without any creative effort, fall into the protection scope of the present invention.

It should be illustrated that, the terms "first" and "second" and the like in the specification, claimsand the above-mentioned drawings of the present invention are used for distinguishing similar objects, and are not necessarily used for describing a specific sequence or precedence order. It should be understood that the sequences used in this way can be interchanged under appropriate circumstances, so that the embodiments of the present invention described herein can be implemented in a sequence other than those illustrated or described herein. Furthermore, the terms "including" and "having", and any variations thereof are intended to cover non-exclusive inclusions, for example, processes, methods, systems, products or devices including a series of steps or units are not necessarily limited to those clearly listed steps or units, but can include other steps or units that are not clearly listed or are inherent to these processes, methods, products or devices.

The embodiments of the present invention can be applied to an electronic device with at least one camera shooting unit, and the electronic device can include: smart phones, tablet computers, electronic readers, desktop computers, workstations, servers, personal digital assistants (PDAs), portable multimedia players (PMP), medical devices, cameras or wearable devices (accessories such as watches, bracelets, glasses and headsets), electronic clothing, skin chips that can be implanted in vivo, vehicle-mounted electronic instruments, etc.

The flowchart of an optional heart rate estimation method according to an embodiment of the present invention will be described below. It should be illustrated that, the steps shown in the flowchart of the drawings can be executed in a computer system, such as a group of computer-executable instructions. Moreover, although a logical sequence is shown in the flowchart, in some cases, the steps shown or described can be executed in a sequence different from that herein.

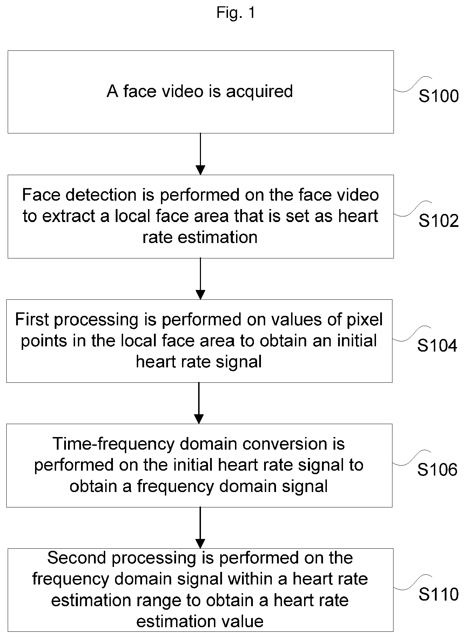

Referring to Fig. 1, it is a flowchart of a first optional heart rate estimation method according to an embodiment of the present invention. As shown in Fig. 1, the heart rate estimation method includes the following steps:
S100: acquiring a face video.

In an optional embodiment, in the present step, the face video is acquired by using a camera shooting apparatus (e.g., an RGB camera, an infrared camera, and so on), and the frame rate is usually greater than 25 fps, so as to keep the succession of a picture, which is conducive to capturing a heart rate state. Whenan infrared camera is utilized, since infrared light is almost invisible tonaked eyes, no influence is generated on human activities; and moreover, the use of the infrared camera can avoid the influence of dark light and complex light, thus having certain anti-interference and anti-blocking capabilities. In addition, because of exact transmitting and receiving wave bands of an infrared light source, a large amount of noise that cannot be avoided due to the use of ordinary RGB camera solutions can be effectively removed, and active light supplementation is realized. The infrared light source can be a built-in light source of the infrared camera, and can also be an external independent light source. The camera shooting apparatus can be an independent camera, or integrated with other cameras in whole or in part to form one or more camera shooting modules, and can be installed independently or installed on the electronic device in an embedded or external manner.

S102: performing face detection on the face video to extract a local face area that is configured to perform heart rate estimation.

In an optional embodiment, in the present step, the local face area that is configured to perform heart rate estimation can also be extracted by combining face key point positioning with face detection. By introducing face key points, it is possible to extract the local face area that is configured to perform heart rate estimation more accurately, so that in the case of face shaking, a heart rate estimation value will not be deviated due to motion blur and facial deformation.

In an optional embodiment, the local face area extracted in the present step includes an area below eyes. Since the area below eyeshas more veins distributed and is not easily blocked compared with other places on the face, by extracting the area below eyes as the local face area, the accuracy of the heart rate estimation method can be improved.

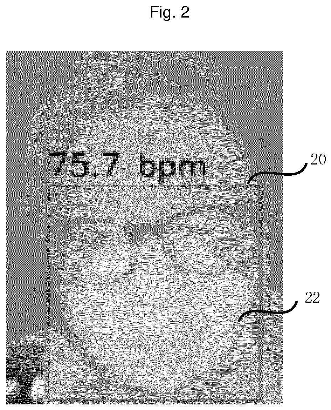

For example, as shown in Fig. 2, it is a schematic diagram of an optional extracted local face area according to an embodiment of the present invention, the local face area that is configured to perform heart rate estimation is extracted by combining face key point positioning with face detection. In Fig. 2, a rectangular frame 20 represents a face area determined by face detection, and an irregular mask frame 22 represents the local face area extracted by the face key points.

Of course, those skilled in the art can be aware that, other areas on the face, such as a forehead anda cheek, can also be extracted as the local face area; or, without creative effort, those skilled in the art can also extract other human body areas with rich veins as detection areas for heart rate estimation, such as a neck anda wrist.

S104: performing first processing on values of pixel points in the local face area to obtain an initial heart rate signal.

The present step further includes: performing weighted average on the values of the pixel points in the local face area according to weights, and taking the value of weighted average as a luminance signal of the current frame; and constituting the initial heart rate signal by the luminance signal of the current frame and luminance signals of previous N historical frames, and N being an integer greater than or equal to 1, wherein the weight is set according to the position of the pixel point in the local face area, and the closer the pixel point is to an edge position in the local face area, the smaller the weight corresponding to the pixel point is. The value range of the weight can be 0-1.

S106: performing time-frequency domain conversion on the initial heart rate signal to obtain a frequency domain signal.

In an optional embodiment, the step of performing time-frequency domain conversion on the initial heart rate signal to obtain the frequency domain signal includes: converting the initial heart rate signal from a time domain into a frequency domain by using fast Fourier transform, to obtain the frequency domain signal.

In an optional embodiment, before performing the time-frequency domain conversion on the initial heart rate signal, the heart rate estimation method can further include: performing first denoising processing on the initial heart rate signal in the time domain, wherein the first denoising processing method includes at least one of the following: S-G (Savitzky-Golay) filtering, detrend (Detrend) filtering, moving average filtering, normalization (Normalize) processing, and bandpass (Bandpass) filtering. The S-G (Savitzky-Golay) filtering and detrend (Detrend) filtering can be configured to reduce the influence of signal baseline translation on the signal (for example, to eliminate the influence of ambient light). The moving average filtering can be configured to remove random noise from the signal. The normalization (Normalize) processingcan facilitate signal processing and improve the computational efficiency. By means of the bandpass (Bandpass) filtering, the signal in a normal heart rate frequency domain band can be processed. By means of the first denoising processing, high-frequency noise, low-frequency noise, and noise introduced by other factors (for example, motion) can be removed, so that the heart rate signal is clearer, and the periodicity is more prominent.

S110: performing second processing on the frequency domain signal within a heart rate estimation range to obtain a heart rate estimation value.

In an optional embodiment, the step of performing second processing on the frequency domain signalto obtain theheart rate estimation value includes:
S1100: performing peak value detection on the frequency domain signal within the heart rate estimation range to obtain peak values;
S1102: sorting the peak values to obtain a sorting result;
S1104: obtaining a confidence coefficient according to the sorting result; and
S1106: obtaining the heart rate estimation value according to the confidence coefficient.

In an optional embodiment, the peak values are sorted in a traversal manner in a descending order of the peak values, and the sorting result is acquired as needed, for example, the highest peak value and a second peak value are selected as the sorting result. A ratio of the highest peak value to the second peak value is taken as the confidence coefficient, that is, confidence coefficient = highest peak value/second peak value.

In another optional embodiment, the step of performing second processing on the frequency domain signalto obtain theheart rate estimation value includes:
S1110: performing peak value detection on the frequency domain signal within the heart rate estimation range to obtain a highest peak value;
S1112: taking the frequency of the highest peak value as main frequency, and calculating the energy of a first-order harmonic and a second-order harmonic corresponding to the main frequency, to obtain an energy calculation value;
S1114: dividing the energy calculation value by the energy of the remaining frequency other than the main frequency, so as to obtain a confidence coefficient; and
S1116: obtaining the heart rate estimation value according to the confidence coefficient.

In an optional embodiment, the heart rate estimation range can be preset, for example, the heart rate estimation range can be preset to 50 beats/min to 180 beats/min according to a heart rate limit value of a human body.

In an optional embodiment, the step of obtaining the heart rate estimation value according to the confidence coefficient includes: comparing the confidence coefficient with a first threshold value to obtain a comparison result; when the confidence coefficientis less than the first threshold value, the comparison result indicates that the frequency domain signal is seriously polluted by noise, then discarding the current frequency domain signal, and detecting the next frame; and when the confidence coefficient is not less than the first threshold value, the comparison result indicates that the frequency domain signal is not polluted by noise or the noise pollution is relatively small, then acquiring the frequency corresponding to the highest peak value as theheart rate estimation value.

In an optional embodiment, before performing second processing on the frequency domain signal, the heart rate estimation method can further include: performing second denoising processing on the frequency domain signal, wherein the second denoising processing method includes at least one or more of the following: discrete Fourier transform (DFT), and bandpass (Bandpass) filtering. By means of the bandpass (Bandpass) filtering, the signal in the normal heart rate frequency domain band can be processed. By performing the second denoising processing on the frequency domain signal, a required principal component signal can be further highlighted.

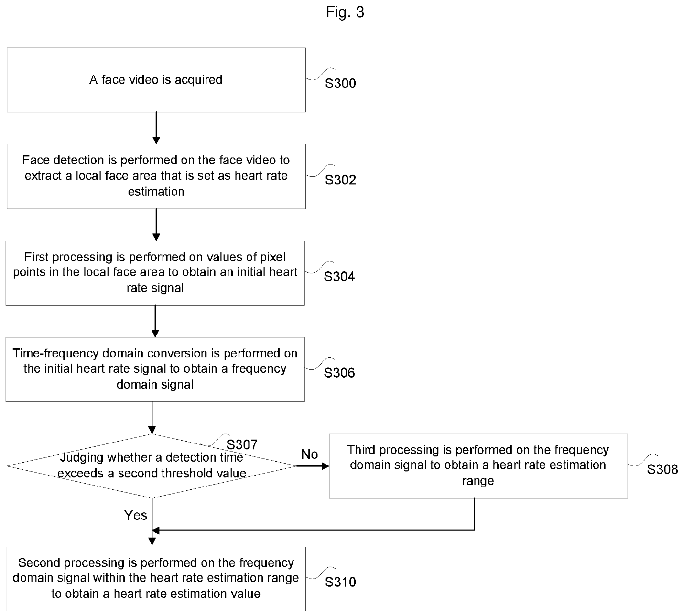

In the heart rate estimation method provided by the above embodiment, if the heart rate estimation range is preset, there may be a case where an output value is unstable due to inappropriate range setting. In order to achieve more accurate and more stable heart rate estimation, the present invention further provides another heart rate estimation method with an automatic range estimation function. Referring to Fig. 3, it is a flowchart of a second optional heart rate estimation method according to an embodiment of the present invention. As shown in Fig. 3, the heart rate estimation method includes the following steps:
S300: acquiring a face video;
S302: performing face detection on the face video to extract a local face area that is configured to perform heart rate estimation;
S304: performing first processing on values of pixel points in the local face area to obtain an initial heart rate signal;
S306: performing time-frequency domain conversion on the initial heart rate signal to obtain a frequency domain signal; and
S307: judging whether a detection time exceeds a second threshold value;
S308: performing third processing on the frequency domain signal to obtain a heart rate estimation range; and
S310: performing second processing on the frequency domain signal within the heart rate estimation range to obtain a heart rate estimation value.

In the heart rate estimation method provided by the present embodiment, the steps S300, S302, S304, S306 and S310 respectively correspond to the steps S100, S102, S104, S106 and S110 in the first embodiment, and thus will not be repeated herein. The difference is that the heart rate estimation method provided by the present embodiment further includes the step S307 and the step S308, which will be described in detail below.

In the step S307, the second threshold value can be set to be M frames or M seconds, M is an integer greater than or equal to 1, the detection time is timed, and a timing value is compared with the second threshold value to judge whether the detection time exceeds the second threshold value; and if the detection time exceeds the second threshold value, the step S307 is skipped to the step S308, and third processing is performed on the frequency domain signal to obtain the heart rate estimation range. The step of performing third processing on the frequency domain signal to obtain the heart rate estimation range can include: continuously selecting consecutive X frames or X seconds of frequency domain signals through a sliding window to obtain a heart rate value, buffering the heart rate value, acquiring the heart rate estimation range by using a deep learning method, repeating the action within M frames or M seconds, and averaging all acquired heart rate estimation ranges to obtain a final heart rate estimation range. If the detection time does not exceed the second threshold value, the step is skipped to the step S310 to perform second processing on the frequency domain signal within the heart rate estimation range, so as to obtain theheart rate estimation value.

According to the heart rate estimation method provided by the embodiment of the present invention, in addition to detecting heart rate estimation, the method can also be used foracquiring cardiovascular parameters such as a respiratory rate, a blood oxygen content (SpO2) and a blood pressure, so as to monitor physical conditions of a person. This heart rate estimation method can be applied to various mobile platforms, vehicle-mounted chips, embedded chips and the like, and it requires no large and complex hardware device, so that the detection process is simple and fast, and no contact and no harm is brought to the human body. At the same time, the method has sufficient accuracy, and solves the problem of the traditional contact detection method of relying on complex hardware device and requiring contact with the human body, therefore the robustness and application range of the heart rate estimation method are greatly improved.

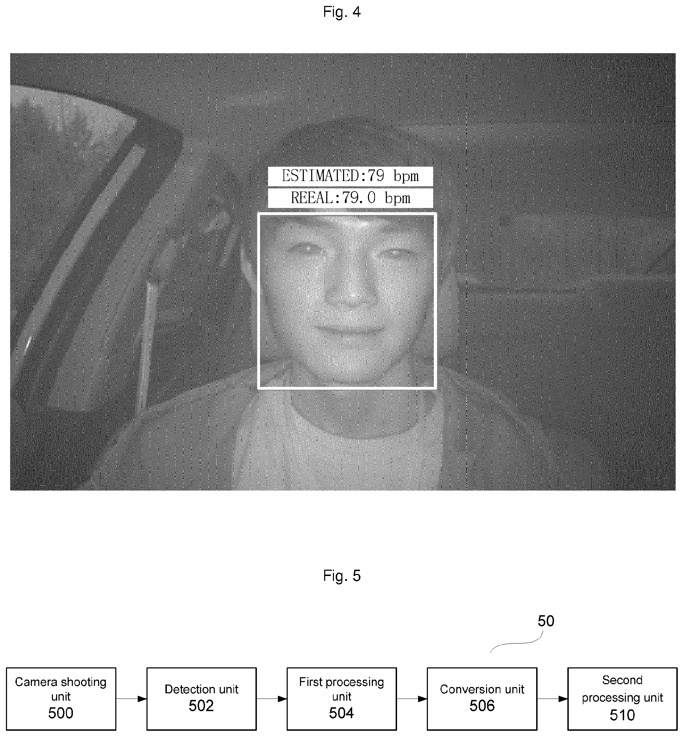

In an application scenario of the embodiment of the present invention, as shown in Fig. 4, it is an application scenario diagram of a heart rate estimation method provided according to an embodiment of the present invention. If the heart rate estimation method is used for monitoring the physical conditions of a driver, the problem can be avoided that a traditional contact heat rate device needs to be worn on the driver, thus generating certain influence on driving. It can be seen from detection results shown in Fig. 4 that, the similarity accuracy of an estimated heart rate value (Estimated) obtained by using the heart rate estimation method and a real heart rate value (Real) is very high. In addition, accurate heart rate estimation can still be realized under dark light conditions such as at nighttime, tunnels and backlight, so as to continuously track and judge the physical conditions of the driver, when it is monitored that the heart rate of the driver is abnormal, an alarm prompt can be given, and an assisted driving function is started. Of course, those skilled in the art can be aware that, the heart rate estimation method provided according to the embodiment of the present invention can also be applied to other scenarios, such as sleep heart rate monitoring.

According to another aspect of the embodiments of the present invention, a heart rate estimation apparatus is further provided. Referring to Fig. 5, it is a structural block diagram of an optional heart rate estimation apparatus according to an embodiment of the present invention. As shown in Fig. 5, the heart rate estimation apparatus 50 includes a camera shooting unit 500, a detection unit 502, a first processing unit 504, a conversion unit 506 and a second processing unit 510.

Each unit included in the heart rate estimation apparatus 50 will be described in detail below.

The camera shooting unit 500 is configured to acquire a face video.

In an optional embodiment, the camera shooting unit 500 can be an RGB camera, an infrared camera, and so on, and the frame rate is usually greater than 25 fps, so as to keep the succession of a picture, which is conducive to capturing a heart rate state. Whenan infrared camera is utilized, since infrared light is almost invisible tonaked eyes, no influence is generated on human activities; and moreover, the use of the infrared camera can avoid the influence of dark light and complex light, thus having certain anti-interference and anti-blocking capabilities. In addition, because of exact transmitting and receiving wave bands of an infrared light source, a large amount of noise that cannot be avoided due to the use of ordinary RGB camera solutions can be effectively removed, and active light supplementation is realized. The infrared light source can be a built-in light source of the infrared camera, and can also be an external independent light source. The camera shooting unit 500 can be an independent camera, or integrated with other cameras in whole or in part to form one or more camera shooting modules, and can be installed independently or installed on an electronic device in an embedded or external manner.

The detection unit 502 is configured to perform face detection on the face video to extract a local face area that is configured to perform heart rate estimation.

In an optional embodiment, in addition to including a face detection module 5020 that is configured to perform face detection, the detection unit 502 can further include a face key point positioning module 5022, which is configured to be combined with face detection module 5020 to extract the local face area that is configured to perform heart rate estimation. By introducing the face key point positioning module 5022, it is possible to extract the local face area that is configured to perform heart rate estimation more accurately, so that in the case of face shaking, a heart rate estimation value will not be deviated due to motion blur and facial deformation.

In an optional embodiment, the local face area extracted in the present step includes an area below eyes. Since the area below eyeshas more veins distributed and is not easily blocked compared with other places on the face, by extracting the area below eyes as the local face area, the accuracy of the heart rate estimation method can be improved.

The first processing unit 504 is configured to perform first processing on values of pixel points in the local face area to obtain an initial heart rate signal.

The first processing unit 504 further includes a luminance signal acquisition module 5040, configured to perform weighted average on the values of the pixel points in the local face area according to weights, and take the value of weighted average as a luminance signal of the current frame; and an initial heart rate signal acquisition module 5042, configured to acquire the initial heart rate signal according to the luminance signal of the current frame and luminance signals of previous N historical frames, and N being an integer greater than or equal to 1, wherein the weight is set according to the position of the pixel point in the local face area, and the closer the pixel point is to an edge position in the local face area, the smaller the weight corresponding to the pixel point is. The value range of the weight can be 0-1.

The conversion unit 506 is configured to perform time-frequency domain conversion on the initial heart rate signal to obtain a frequency domain signal.

In an optional embodiment, the conversion unit 506 can convert the initial heart rate signal from a time domain into a frequency domain by using fast Fourier transform, to obtain the frequency domain signal.

In an optional embodiment, the heart rate estimation apparatus 50can further include: a first denoising module configured to, before performing time-frequency domain conversion on the initial heart rate signal, perform first denoising processing on the initial heart rate signal in the time domain, wherein the first denoising processing method includes at least one of the following: S-G (Savitzky-Golay) filtering, detrend (Detrend) filtering, moving average filtering, normalization (Normalize) processing, and bandpass (Bandpass) filtering. The S-G (Savitzky-Golay) filtering and detrend (Detrend) filtering can be configured to reduce the influence of signal baseline translation on the signal (for example, to eliminate the influence of ambient light). The moving average filtering can be configured to remove random noise from the signal. The normalization (Normalize) processingcan facilitate signal processing and improve the computational efficiency. By means of the bandpass (Bandpass) filtering, the signal in a normal heart rate frequency domain band can be processed. By means of the first denoising processing, high-frequency noise, low-frequency noise, and noise introduced by other factors (for example, motion) can be removed, so that the heart rate signal is clearer, and the periodicity is more prominent.

The second processing unit 510 is configured to performsecond processing on the frequency domain signal within a heart rate estimation range to obtain a heart rate estimation value.

In an optional embodiment, the second processing unit 510 includes:
a peak detection module 5100, configured toperform peak value detection on the frequency domain signal within the heart rate estimation range to obtain peak values;
a sorting module 5102, configured to sort the peak values to obtain a sorting result;
a confidence coefficient calculation module 5104, configured to calculate a confidence coefficient according to the sorting result; and
an estimation module 5106, configured to obtain the heart rate estimation value according to the confidence coefficient.

In an optional embodiment, the peak values are sorted in a traversal manner in a descending order of the peak values, and the sorting result is acquired as needed, for example, the highest peak value and a second peak value are selected as the sorting result. A ratio of the highest peak value to the second peak value is taken as the confidence coefficient, that is, confidence coefficient = highest peak value/second peak value.

In anoptional embodiment, the second processing unit 510 includes:
a highest peak detection module 5110, configured to perform peak value detection on the frequency domain signal within the heart rate estimation range to obtain a highest peak value;
an energy calculation module 5112, configured to take the frequency of the highest peak value as main frequency, and calculate the energy of a first-order harmonic and a second-order harmonic corresponding to the main frequency, to obtain an energy calculation value;
a confidence coefficient calculation module 5114, configured todivide the energy calculation value by the energy of the remaining frequency other than the main frequency, to obtain a confidence coefficient; and
an estimation module 5116, configured to obtain the heart rate estimation value according to the confidence coefficient.

In an optional embodiment, the heart rate estimation range can be preset, for example, the heart rate estimation range can be preset to 50 beats/min to 240 beats/min according to a heart rate limit value of a human body.

In an optional embodiment, the estimation module 5106 is configured to compare the confidence coefficient with a first threshold value to obtain a comparison result; when the confidence coefficientis less than the first threshold value, the comparison result indicates that the frequency domain signal is seriously polluted by noise, then discard the current frequency domain signal, and detect the next frame; and when the confidence coefficient is not less than the first threshold value, the comparison result indicates that the frequency domain signal is not polluted by noise or the noise pollution is relatively small, then acquire the frequency corresponding to the highest peak value as theheart rate estimation value.

In an optional embodiment, the heart rate estimation apparatus 50 can further include a second denoising module configured to, before performing second processing on the frequency domain signal, perform second denoising processing on the frequency domain signal, wherein the second denoising processing method includes at least one or more of the following: discrete Fourier transform (DFT), and bandpass (Bandpass) filtering. By means of the bandpass (Bandpass) filtering, the signal in the normal heart rate frequency domain band can be processed. By performing the second denoising processing on the frequency domain signal, a required principal component signal can be further highlighted.

In another optional embodiment, the heart rate estimation apparatus 50 can further include a judging module 507, configured to judge whether a detection time exceeds a second threshold value, wherein the second threshold value can be set to be M frames or M seconds, M is an integer greater than or equal to 1, the detection time is timed, and a timing value is compared with the second threshold value to judge whether the detection time exceeds the second threshold value; and a third processing module 508 configured to, when the detection time exceeds the second threshold value, perform third processing on the frequency domain signal to obtain the heart rate estimation range, wherein the step of performing third processing on the frequency domain signal to obtain the heart rate estimation range can include: continuously selecting consecutive X frames or X seconds of frequency domain signals through a sliding window to obtain a heart rate value, buffering the heart rate value, acquiring the heart rate estimation range by using a deep learning method, repeating the action within M frames or M seconds, and averaging all acquired heart rate estimation ranges to obtain a final heart rate estimation range. If the detection time does not exceed the second threshold value, the second processing unit 510 performs second processing on the frequency domain signal within the heart rate estimation range to obtain theheart rate estimation value.

According to another aspect of the embodiments of the present invention, an electronic device is further provided, including: a processor; and a memory, configured to store executable instructions of the processor, wherein the processor is configured to execute the heart rate estimation method in any of above embodiments by executing the executable instructions.

According to another aspect of the embodiments of the present invention, a storage medium is further provided, including a stored program, wherein the program, when running, controls a device where the storage medium is located to execute the heart rate estimation method in any of above embodiments.

The serial numbers of the above embodiments of the present invention are only for description, and do not represent the advantages or disadvantages of the embodiments.

In the above embodiments of the present invention, the description of each embodiment has its own emphasis. For parts that are not described in detail in a certain embodiment, reference can be made to related descriptions of other embodiments.

In the several embodiments provided by the present application, it should be understood that, the disclosed technical content can be implemented in other manners. The apparatus embodiments described above are merely exemplary, for example, the division of the units is only a logic function division, there can be other division manners in practical implementation, for example, a plurality of units or components can be combined or integrated to another system, or some features can be omitted or not implemented. From another point of view, the displayed or discussed mutual coupling or direct coupling or communication connection can be indirect coupling or communication connection of units or modules through some interfaces, and can be in electrical, mechanical or other forms.

The units described as separate components can be separated physically or not, components displayed as units can be physical units or not, namely, can be located in one place, or can be distributed on a plurality of units. A part of or all of the units can be selected to implement the purposes of the solutions in the present embodiment according to actual demands.

In addition, the functional units in various embodiments of the present invention can be integrated in a processing unit, or the units individually exist physically, or two or more units are integrated in one unit. The integrated unit can be implemented in the form of hardware, and can also be implemented in the form of a software functional unit.

If the integrated unit is implemented in the form of the software functional unit and is sold or used as an independent product, it can be stored in a computer-readable storage medium. Based on this understanding, the technical solutions of the present invention substantially, or the part contributing to the prior art, or part of or all the technical solutions can be implemented in the form of a software product, the computer software product is stored in a storage medium, and includes several instructions for enabling a computer device (which can be a personnel computer, a server, or a network device or the like) to execute all or part of the steps of the method in various embodiments of the present invention. The foregoing storage medium includes a variety of media capable of storing program codes, such as a USB disk, a read-only memory (ROM, Read-Only Memory), a random access memory (RAM, Random Access Memory), a mobile hard disk, a magnetic disk, or an optical disk.

The foregoing descriptions are merely specific embodiments of the present invention. The scope of the invention is set out in the appended claims.

### Industrial Applicability

The solutions provided by the embodiments of the present application can monitor the physical conditions of a person. The technical solutions provided by the embodiments of the present application can be applied to an electronic device with at least one camera shooting unit, for example, applicable to various mobile platforms, vehicle-mounted chips, embedded chips and the like, and it requires no large and complex hardware device, so that the detection process is simple and fast, and no contact and no harm is brought to the human body. At the same time, the accuracy is enough, the problem of a traditional contact detection method of relying on complex hardware device and requiring contact with the human body is solved, and the robustness and application range of the heart rate estimation method are greatly improved. The physical conditions of a driver are continuously tracked and judged, when it is monitored that the heart rate of the driver is abnormal, an alarm prompt can be given, and an assisted driving function is started.

## Claims

1. A computer-implemented heart rate estimation method, comprising:
acquiring (S100) a face video;
performing (S102) face detection on the face video to extract a local face area that is configured to perform heart rate estimation;
performing (S104) first processing on values of pixel points in the local face area to obtain an initial heart rate signal;
performing (S106) time-frequency domain conversion on the initial heart rate signal to obtain a frequency domain signal; and
performing (S110) second processing on the frequency domain signal within a heart rate estimation range to obtain a heart rate estimation value;
**characterized in that** the step of performing first processing on the values of the pixel points in the local face area to obtain the initial heart rate signal comprises: performing weighted average on the values of the pixel points in the local face area according to weights, and taking the value of weighted average as a luminance signal of the current frame; and constituting the initial heart rate signal by the luminance signal of the current frame and luminance signals of previous N historical frames;
and **in that** the weight is set according to the position of the pixel point in the local face area, and the closer the pixel point is to an edge position in the local face area, the smaller the weight corresponding to the pixel point is.

2. The heart rate estimation method as claimed in claim 1, further comprising combining face key point positioning with face detection to extract the local face area that is configured to perform heart rate estimation.

3. The heart rate estimation method as claimed in claim 1, wherein before performing the time-frequency domain conversion on the initial heart rate signal, the heart rate estimation method further comprises performing first denoising processing on the initial heart rate signal in the time domain, wherein the first denoising processing method includes at least one of the following: Savitzky-Golay filtering, detrend filtering, moving average filtering, normalization processing, and bandpass filtering.

4. The heart rate estimation method as claimed in claim 1, wherein the step of performing second processing on the frequency domain signal within the heart rate estimation range to obtain the heart rate estimation value comprises:
performing peak value detection on the frequency domain signal within the heart rate estimation range to obtain peak values;
sorting the peak values to obtain a sorting result;
obtaining a confidence coefficient according to the sorting result; and
obtaining the heart rate estimation value according to the confidence coefficient.

5. The heart rate estimation method as claimed in claim 1, wherein the step of performing second processing on the frequency domain signal within the heart rate estimation range to obtain the heart rate estimation value comprises:
performing peak value detection on the frequency domain signal within the heart rate estimation range to obtain a highest peak value;
taking the frequency of the highest peak value as main frequency, and calculating the energy of a first-order harmonic and a second-order harmonic corresponding to the main frequency, to obtain an energy calculation value;
dividing the energy calculation value by the energy of the remaining frequency other than the main frequency, to obtain a confidence coefficient; and
obtaining the heart rate estimation value according to the confidence coefficient.

6. The heart rate estimation method as claimed in claim 5, wherein the heart rate estimation method further comprises:
judging whether a detection time exceeds a second threshold value; and
when the detection time exceeds the second threshold value, performing third processing on the frequency domain signal to obtain the heart rate estimation range.

7. The heart rate estimation method as claimed in claim 6, wherein the step of performing third processing on the frequency domain signal to obtain the heart rate estimation range comprises: continuously selecting consecutive X frames or X seconds of frequency domain signals through a sliding window to obtain a heart rate value, buffering the heart rate value, acquiring the heart rate estimation range by using a deep learning method, repeating the action within M frames or M seconds, and averaging all acquired heart rate estimation ranges to obtain a final heart rate estimation range.

8. The heart rate estimation method as claimed in claim 4, wherein the peak values are sorted in a traversal manner in a descending order of the peak values, the highest peak value and a second peak value are selected as the sorting result, and a ratio of the highest peak value to the second peak value is taken as the confidence coefficient.

9. The heart rate estimation method as claimed in claim 6, wherein the step of obtaining the heart rate estimation value according to the confidence coefficient comprises:
comparing the confidence coefficient with a first threshold value to obtain a comparison result;
when the confidence coefficient is less than the first threshold value, the comparison result indicates that the frequency domain signal is seriously polluted by noise, then discarding the current result, and detecting the next frame; and
when the confidence coefficient is not less than the first threshold value, the comparison result indicates that the frequency domain signal is not polluted by noise or the noise pollution is relatively small, then acquiring the frequency corresponding to the highest peak value as the heart rate estimation value.

10. The heart rate estimation method as claimed in claim 1, wherein before performing second processing on the frequency domain signal, the heart rate estimation method further comprises: performing second denoising processing on the frequency domain signal, wherein the second denoising processing method comprises at least one or more of the following: discrete Fourier transform (DFT), and bandpass (Bandpass) filtering.

11. A heart rate estimation apparatus (50) comprising:
a camera shooting unit (500) configured to acquire a face video;
a detection unit (502) configured to perform face detection on the face video to extract a local face area that is configured to perform heart rate estimation;
a first processing unit (504) configured to perform first processing on values of pixel points in the local face area to obtain an initial heart rate signal;
a conversion unit (506) configured to perform time-frequency domain conversion on the initial heart rate signal to obtain a frequency domain signal; and
a second processing unit (510) configured to perform second processing on the frequency domain signal within a heart rate estimation range to obtain a heart rate estimation value;
**characterized in that** the first processing unit is further configured to:
- perform (5040) weighted average on the values of the pixel points in the local face area according to weights,
- take the value of weighted average as a luminance signal of the current frame; and
- constitute (5042) the initial heart rate signal by the luminance signal of the current frame and luminance signals of previous N historical frames;
wherein the weight is set according to the position of the pixel point in the local face area, and the closer the pixel point is to an edge position in the local face area, the smaller the weight corresponding to the pixel point is.

12. A storage medium comprising a stored program, and the program, when running, controls a device where the storage medium is located to execute the heart rate estimation method as claimed in any of claims 1-10.

13. An electronic device comprising:
a processor; and
a memory configured to store executable instructions of the processor,
wherein the processor is configured to execute the heart rate estimation method as claimed in any of claims 1-10 by executing the executable instructions .

## Patentansprüche

1. Computerimplementiertes Verfahren zur Kalkulation der Herzfrequenz, umfassend:
Erfassen (S100) eines Gesichtsvideos;
Durchführen (S102) einer Gesichtserkennung auf dem Gesichtsvideo, um eine lokale Gesichtsregion zu extrahieren, die ausgelegt ist, um die Herzfrequenzkalkulation durchzuführen;
Durchführen (S104) einer ersten Verarbeitung auf Werten von Pixelpunkten in der lokalen Gesichtsregion, um ein anfängliches Herzfrequenzsignal zu erhalten;
Durchführen (S106) einer Zeit-Frequenzbereich-Umwandlung auf dem anfänglichen Herzfrequenzsignal, um ein Frequenzbereichssignal zu erhalten, und
Durchführen (S110) einer zweiten Verarbeitung auf dem Frequenzbereichssignal innerhalb eines Herzfrequenzkalkulationsbereichs, um einen Herzfrequenzkalkulationswert zu erhalten,
**dadurch gekennzeichnet, dass** der Schritt zum Durchführen einer ersten Verarbeitung auf den Werten der Pixelpunkte in der lokalen Gesichtsregion, um das anfängliche Herzfrequenzsignal zu erhalten, Folgendes umfasst:
Durchführen eines gewichteten Durchschnitts auf den Werten der Pixelpunkte in der lokalen Gesichtsregion nach Gewichtungen und Heranziehen des gewichteten Durchschnittswerts als Leuchtdichtensignal des aktuellen Bildfelds und Bilden des anfänglichen Herzfrequenzsignals durch das Leuchtdichtensignal des aktuellen Bildfelds und der Leuchtdichtensignale von vorherigen N historischen Bildfeldern,
und dadurch, dass die Gewichtung gemäß der Position des Pixelpunkts in der lokalen Gesichtsregion festgelegt wird, und je näher sich der Pixelpunkt an einer Randposition in der lokalen Gesichtsregion befindet, desto kleiner ist die dem Pixelpunkt entsprechende Gewichtung.

2. Verfahren zur Kalkulation der Herzfrequenz nach Anspruch 1, ferner umfassend das Kombinieren von Gesichtsschlüsselpunktpositionieren mit Gesichtserkennung, um die lokale Gesichtsregion zu extrahieren, die ausgelegt ist, um die Herzfrequenzkalkulation durchzuführen.

3. Verfahren zur Kalkulation der Herzfrequenz nach Anspruch 1, wobei das Verfahren zur Kalkulation der Herzfrequenz vor dem Durchführen der Zeit-Frequenzbereich-Umwandlung auf dem anfänglichen Herzfrequenzsignal ferner das Durchführen einer ersten Entrauschungsverarbeitung auf dem anfänglichen Herzfrequenzsignal im Zeitbereich umfasst, wobei das Verfahren zur ersten Entrauschungsverarbeitung mindestens einen der folgenden Vorgänge einschließt: Savitzky-Golay-Filterung, Trendbereinigungsfilterung, Gleitender-Mittelwert-Filterung, Normalisierungsverarbeitung und Bandpass-Filterung.

4. Verfahren zur Kalkulation der Herzfrequenz nach Anspruch 1, wobei der Schritt zum Durchführen einer zweiten Verarbeitung auf dem Frequenzbereichssignal innerhalb des Herzfrequenzkalkulationsbereichs, um den Herzfrequenzkalkulationswert zu erhalten, Folgendes umfasst:
Durchführen einer Spitzenwerterkennung auf dem Frequenzbereichssignal innerhalb des Herzfrequenzkalkulationsbereichs, um Spitzenwerte zu erhalten;
Sortieren der Spitzenwerte, um ein Sortierergebnis zu erhalten;
Erhalten eines Konfidenzkoeffizienten je nach dem Sortierergebnis, und
Erhalten des Herzfrequenzkalkulationswerts je nach dem Konfidenzkoeffizienten.

5. Verfahren zur Kalkulation der Herzfrequenz nach Anspruch 1, wobei der Schritt zum Durchführen einer zweiten Verarbeitung auf dem Frequenzbereichssignal innerhalb des Herzfrequenzkalkulationsbereichs, um den Herzfrequenzkalkulationswert zu erhalten, Folgendes umfasst:
Durchführen einer Spitzenwerterkennung auf dem Frequenzbereichssignal innerhalb des Herzfrequenzkalkulationsbereichs, um einen höchsten Spitzenwert zu erhalten;
Heranziehen der Frequenz des höchsten Spitzenwerts als Hauptfrequenz und Berechnen der Energie einer Harmonischen erster Ordnung und einer Harmonischen zweiter Ordnung entsprechend der Hauptfrequenz, um einen Energieberechnungswert zu erhalten;
Trennen des Energieberechnungswerts durch die Energie der Restfrequenz, bei der es sich nicht um die Hauptfrequenz handelt, um einen Konfidenzkoeffizienten zu erhalten, und
Erhalten des Herzfrequenzkalkulationswerts je nach dem Konfidenzkoeffizienten.

6. Verfahren zur Kalkulation der Herzfrequenz nach Anspruch 5, wobei das Verfahren zur Kalkulation der Herzfrequenz ferner Folgendes umfasst:
Beurteilen, ob eine Erkennungszeit einen zweiten Schwellenwert überschreitet, und
Durchführen einer dritten Verarbeitung auf dem Frequenzbereichssignal, um den Herzfrequenzkalkulationsbereich zu erhalten, wenn die Erkennungszeit den zweiten Schwellenwert überschreitet.

7. Verfahren zur Kalkulation der Herzfrequenz nach Anspruch 6, wobei der Schritt zum Durchführen der dritten Verarbeitung auf dem Frequenzbereichssignal, um den Herzfrequenzkalkulationsbereich zu erhalten, Folgendes umfasst:
kontinuierliches Auswählen aufeinanderfolgender X Bildfelder oder X Sekunden von Frequenzbereichssignalen durch ein Schiebefenster, um einen Herzfrequenzwert zu erhalten, Speichern des Herzfrequenzwerts, Erfassen des Herzfrequenzkalkulationsbereichs durch Nutzen eines Deep-Learning-Verfahrens, Wiederholen des Vorgangs innerhalb von M Bildfeldern oder M Sekunden, und Mitteln aller erfassten Herzfrequenzkalkulationsbereiche, um einen finalen Herzfrequenzkalkulationsbereich zu erhalten.

8. Verfahren zur Kalkulation der Herzfrequenz nach Anspruch 4, wobei die Spitzenwerte quer in absteigender Reihenfolge der Spitzenwerte sortiert werden, der höchste Spitzenwert und ein zweiter Spitzenwert als Sortierergebnis ausgewählt werden und ein Verhältnis des höchsten Spitzenwerts zum zweiten Spitzenwert als Konfidenzkoeffizient herangezogen wird.

9. Verfahren zur Kalkulation der Herzfrequenz nach Anspruch 6, wobei der Schritt zum Erhalten des Herzfrequenzkalkulationswerts je nach dem Konfidenzkoeffizienten Folgendes umfasst:
Vergleichen des Konfidenzkoeffizienten mit einem ersten Schwellenwert, um ein Vergleichsergebnis zu erhalten;
wenn der Konfidenzkoeffizient kleiner ist als der erste Schwellenwert, gibt das Vergleichsergebnis an, dass das Frequenzbereichssignal schwerwiegend durch Rauschen beeinträchtigt ist, dann Aussortieren des aktuellen Ergebnisses und Erkennen des nächsten Bildfelds, und
wenn der Konfidenzkoeffizient nicht kleiner ist als der erste Schwellenwert, gibt das Vergleichsergebnis an, dass das Frequenzbereichssignal nicht durch Rauschen beeinträchtigt ist oder die Belastung durch Rauschen relativ gering ist, dann Erfassen der Frequenz, die dem höchsten Spitzenwert entspricht, als Herzfrequenzkalkulationswert.

10. Verfahren zur Kalkulation der Herzfrequenz nach Anspruch 1, wobei das Verfahren zur Kalkulation der Herzfrequenz vor dem Durchführen der zweiten Verarbeitung auf dem Frequenzbereichssignal ferner Folgendes umfasst:
Durchführen einer zweiten Entrauschungsverarbeitung auf dem Frequenzbereichssignal, wobei das Verfahren zur zweiten Entrauschungsverarbeitung mindestens einen oder mehrere der folgenden Vorgänge umfasst: diskrete Fourier-Transformation (OFT) und Bandpass-Filterung (Bandpass).

11. Vorrichtung zur Kalkulation der Herzfrequenz (50), umfassend:
eine Kameraaufnahmeeinheit (500), die ausgelegt ist, um ein Gesichtsvideo zu erfassen;
eine Erkennungseinheit (502), die ausgelegt ist, um eine Gesichtserkennung auf dem Gesichtsvideo durchzuführen, um eine lokale Gesichtsregion zu extrahieren, die ausgelegt ist, um die Herzfrequenzkalkulation durchzuführen;
eine erste Verarbeitungseinheit (504), die ausgelegt ist, um eine erste Verarbeitung auf Werten von Pixelpunkten in der lokalen Gesichtsregion durchzuführen, um ein anfängliches Herzfrequenzsignal zu erhalten;
eine Umwandlungseinheit (506), die ausgelegt ist, um eine Zeit-Frequenzbereich-Umwandlung auf dem anfänglichen Herzfrequenzsignal durchzuführen, um ein Frequenzbereichssignal zu erhalten, und
eine zweite Verarbeitungseinheit (510), die ausgelegt ist, um eine zweite Verarbeitung auf dem Frequenzbereichssignal innerhalb eines Herzfrequenzkalkulationsbereichs durchzuführen, um einen Herzfrequenzkalkulationswert zu erhalten,
**dadurch gekennzeichnet, dass** die erste Verarbeitungseinheit ferner ausgelegt ist, um
- den gewichteten Durchschnitt bezüglich der Werte der Pixelpunkte in der lokalen Gesichtsregion nach Gewichtungen durchzuführen (5040);
- den gewichteten Durchschnittswert als Leuchtdichtesignal des aktuellen Bildfelds heranzuziehen, und
- das anfängliche Herzfrequenzsignal durch das Leuchtdichtesignal des aktuellen Bildfelds und die Leuchtdichtesignale von vorherigen N historischen Bildfeldern zu bilden (5042);
wobei die Gewichtung gemäß der Position des Pixelpunkts in der lokalen Gesichtsregion festgelegt wird, und je näher sich der Pixelpunkt an einer Randposition in der lokalen Gesichtsregion befindet, desto kleiner ist die dem Pixelpunkt entsprechende Gewichtung.

12. Speichermedium, umfassend ein gespeichertes Programm, wobei das Programm, wenn es ausgeführt wird, ein Gerät steuert, in dem das Speichermedium befindlich ist, um das Verfahren zur Kalkulation der Herzfrequenz nach den Ansprüchen 1 bis 10 durchzuführen.

13. Elektronisches Gerät, umfassend:
einen Prozessor, und
einen Speicher, der ausgelegt ist, um ausführbare Anweisungen des Prozessors zu speichern,
wobei der Prozessor ausgelegt ist, um das Verfahren zur Kalkulation der Herzfrequenz nach einem der Ansprüche 1 bis 10 auszuführen, indem die ausführbaren Anweisungen ausgeführt werden.

## Revendications

1. Procédé d'estimation de fréquence cardiaque mis en œuvre par ordinateur, comprenant :
acquérir (S100) une vidéo du visage ;
effectuer (S102) une détection du visage sur la vidéo du visage pour extraire une zone du visage locale qui est configurée pour effectuer une estimation de fréquence cardiaque ;
effectuer (S104) un premier traitement sur des valeurs de points de pixel dans la zone locale du visage pour obtenir un signal de fréquence cardiaque initial ;
effectuer (S106) une conversion de domaine temps-fréquence sur le signal de fréquence cardiaque initial pour obtenir un signal de domaine de fréquence ; et
effectuer (S110) un second traitement sur le signal du domaine de fréquence dans une plage d'estimation de fréquence cardiaque pour obtenir une valeur d'estimation de fréquence cardiaque ;
**caractérisé en ce que** l'étape consistant à effectuer un premier traitement sur les valeurs des points de pixel dans la zone du visage local pour obtenir le signal de fréquence cardiaque initial comprend : réaliser une moyenne pondérée sur les valeurs des points de pixel dans la zone du visage local en fonction des poids, et la prise de la valeur de la moyenne pondérée en tant que signal de luminance de la trame actuelle ; et la constitution du signal de fréquence cardiaque initial par le signal de luminance de la trame actuelle et les signaux de luminance des N trames historiques précédentes ;
et **en ce que** le poids est fixé en fonction de la position du point de pixel dans la zone du visage locale, et plus le point de pixel est proche d'une position de bord dans la zone du visage locale, plus le poids correspondant au point de pixel est petit.

2. Procédé d'estimation de fréquence cardiaque selon la revendication 1, comprenant en outre combiner le positionnement du point clé du visage avec la détection du visage pour extraire la zone locale du visage qui est configurée pour effectuer une estimation de fréquence cardiaque.

3. Procédé d'estimation de fréquence cardiaque selon la revendication 1, dans lequel avant d'effectuer la conversion de domaine temps-fréquence sur le signal de fréquence cardiaque initial, le procédé d'estimation de fréquence cardiaque comprend en outre l'exécution d'un premier traitement de débruitage sur le signal de fréquence cardiaque initial dans le domaine temporel, dans lequel le premier procédé de traitement de débruitage inclut au moins l'un des éléments suivants : filtrage Savitzky-Golay, filtrage de tendance, filtrage de moyenne mobile, traitement de normalisation et filtrage passe-bande.

4. Procédé d'estimation de fréquence cardiaque selon la revendication 1, dans lequel l'étape consistant à effectuer un second traitement sur le signal du domaine fréquentiel dans la plage d'estimation de fréquence cardiaque pour obtenir la valeur d'estimation de fréquence cardiaque comprend :
effectuer une détection de valeur de crête sur le signal du domaine fréquentiel dans la plage d'estimation de fréquence cardiaque pour obtenir des valeurs de crête ;
trier les valeurs de crête pour obtenir un résultat de tri ;
obtenir un coefficient de confiance en fonction du résultat du tri ; et
obtenir la valeur d'estimation de fréquence cardiaque en fonction du coefficient de confiance.

5. Procédé d'estimation de fréquence cardiaque selon la revendication 1, dans lequel l'étape consistant à effectuer un second traitement sur le signal du domaine fréquentiel dans la plage d'estimation de fréquence cardiaque pour obtenir la valeur d'estimation de fréquence cardiaque comprend :
effectuer une détection de valeur de crête sur le signal du domaine fréquentiel dans la plage d'estimation de fréquence cardiaque pour obtenir une valeur de crête la plus élevée ;
prendre la fréquence de la valeur de crête la plus élevée comme fréquence principale, et calculer l'énergie d'une harmonique du premier ordre et d'une harmonique du second ordre correspondant à la fréquence principale, pour obtenir une valeur de calcul d'énergie ;
diviser la valeur de calcul de l'énergie par l'énergie de la fréquence restante autre que la fréquence principale, pour obtenir un coefficient de confiance ; et
obtenir la valeur d'estimation de fréquence cardiaque en fonction du coefficient de confiance.

6. Procédé d'estimation de fréquence cardiaque selon la revendication 5, dans lequel le procédé d'estimation de fréquence cardiaque comprend en outre :
juger si un temps de détection dépasse une deuxième valeur de seuil ; et
lorsque le temps de détection dépasse la deuxième valeur de seuil, effectuer un troisième traitement sur le signal du domaine fréquentiel pour obtenir la plage d'estimation de fréquence cardiaque.

7. Procédé d'estimation de fréquence cardiaque selon la revendication 6, dans lequel l'étape consistant à effectuer un troisième traitement sur le signal du domaine fréquentiel pour obtenir la plage d'estimation de fréquence cardiaque comprend :
sélectionner en continu X trames consécutives ou X secondes de signaux de domaine fréquentiel à travers une fenêtre glissante pour obtenir une valeur de fréquence cardiaque, mettre en mémoire tampon la valeur de fréquence cardiaque, acquérir la plage d'estimation de fréquence cardiaque en utilisant une méthode d'apprentissage en profondeur, répéter l'action dans M trames ou M secondes, et faire la moyenne de toutes les plages d'estimation de fréquence cardiaque acquises pour obtenir une plage d'estimation de fréquence cardiaque finale.

8. Procédé d'estimation de fréquence cardiaque selon la revendication 4, dans lequel les valeurs de crête sont triées de manière transversale dans un ordre décroissant des valeurs de crête, la valeur de crête la plus élevée et une seconde valeur de crête sont sélectionnées en tant que résultat de tri, et un rapport de la valeur de crête la plus élevée à la seconde valeur de crête est pris en tant que coefficient de confiance.

9. Procédé d'estimation de fréquence cardiaque selon la revendication 6, dans lequel l'étape consistant à obtenir la valeur d'estimation de fréquence cardiaque selon le coefficient de confiance comprend :
comparer le coefficient de confiance à une première valeur de seuil pour obtenir un résultat de comparaison ;
lorsque le coefficient de confiance est inférieur à la première valeur de seuil, le résultat de la comparaison indique que le signal du domaine de fréquence est gravement pollué par le bruit, puis écarter le résultat actuel et détecter la trame suivante ; et
lorsque le coefficient de confiance n'est pas inférieur à la première valeur de seuil, le résultat de la comparaison indique que le signal du domaine fréquentiel n'est pas pollué par le bruit ou que la pollution sonore est relativement faible, puis acquérir la fréquence correspondant à la valeur de crête la plus élevée en tant que valeur d'estimation de fréquence cardiaque.

10. Procédé d'estimation de fréquence cardiaque selon la revendication 1, dans lequel avant d'effectuer un second traitement sur le signal du domaine fréquentiel, le procédé d'estimation de fréquence cardiaque comprend en outre :
effectuer un second traitement de débruitage sur le signal du domaine de fréquence, dans lequel le second procédé de traitement de débruitage comprend au moins un ou plusieurs des éléments suivants : la transformation de Fourier discrète (DFT) et le filtrage passe-bande (Bandpass).

11. Appareil d'estimation de fréquence cardiaque (50), comprenant :
une unité de prise de vue par caméra (500) configurée pour acquérir une vidéo du visage ;
une unité de détection (502) configurée pour effectuer une détection du visage sur la vidéo du visage pour extraire une zone du visage locale qui est configurée pour effectuer une estimation de fréquence cardiaque ;
une première unité de traitement (504) configurée pour effectuer un premier traitement sur des valeurs de points de pixel dans la zone locale du visage pour obtenir un signal de fréquence cardiaque initial ;
une unité de conversion (506) configurée pour effectuer une conversion de domaine temps-fréquence sur le signal de fréquence cardiaque initial pour obtenir un signal de domaine fréquentiel ; et
une seconde unité de traitement (510) configurée pour effectuer un second traitement sur le signal du domaine fréquentiel dans une plage d'estimation de fréquence cardiaque pour obtenir une valeur d'estimation de fréquence cardiaque ;
**caractérisé en ce que** la première unité de traitement est en outre configurée pour :
- effectuer (5040) une moyenne pondérée sur les valeurs des points pixels dans la zone du visage local en fonction des poids,
- prendre la valeur de la moyenne pondérée comme signal de luminance de la trame courante ; et
- constituer (5042) le signal de fréquence cardiaque initial par le signal de luminance de la trame courante et les signaux de luminance des N trames historiques précédentes ;
dans lequel le poids est fixé en fonction de la position du point de pixel dans la zone du visage locale, et plus le point de pixel est proche d'une position de bord dans la zone du visage locale, plus le poids correspondant au point de pixel est petit.

12. Support de stockage comprenant un programme stocké, et le programme, lorsqu'il est en cours d'exécution, commande un dispositif où le support de stockage est situé pour exécuter le procédé d'estimation de fréquence cardiaque selon l'une quelconque des revendications 1-10.

13. Dispositif électronique, comprenant :
un processeur ; et
une mémoire configurée pour stocker des instructions exécutables du processeur,
dans lequel le processeur est configuré pour exécuter le procédé d'estimation de fréquence cardiaque selon l'une quelconque des revendications 1-10 en exécutant les instructions exécutables.
